# EUROPÄISCHE PATENTSCHRIFT

(11) **EP 0 324 945 B1**
(45) Veröffentlichungstag und Bekanntmachung des Hinweises auf die Patenterteilung: **09.03.1994**
(21) Anmeldenummer: 88120776.5
(22) Anmeldetag: 13.12.1988
(51) Int. Cl.: C07D 471/08, C07D 519/00, C08K 5/34

(54) **Heterocyclische Bicyclo-[3.3.1]nonanderivate und deren Verwendung**
Heterocyclic bicyclo [3,3,1] nonane derivatives and their use
Dérivés hétérocycliques de bicyclo [3.3.1] nonane et leur application

(30) Priorität: 19.12.1987 DE 3743279
(43) Veröffentlichungstag der Anmeldung: 26.07.1989
(73) Patentinhaber: BASF Aktiengesellschaft, 67063 Ludwigshafen (DE)
(72) Erfinder: Aumueller, Alexander, Dr., D-6705 Deidesheim (DE); Neumann, Peter, Dr., D-6800 Mannheim 31 (DE); Trauth, Hubert, D-6724 Dudenhofen (DE)

(56) Entgegenhaltungen:
- EP-A- 0 213 570
- EP-A- 0 272 590
- JOURNAL OF THE CHEMICAL SOCIETY, Teil II, 1951, Seiten 1706-1708, The Chemical Society, London, GB; Z.Y. KYI et al.: "Synthetic analgesics and related compounds. Part II. Some derivatives of 3 : 7-diazabicyclo[3 : 3 : 1]nonane (Bispidine)

## Beschreibung

Es ist bekannt, daß Polyalkylpiperidinderivate und sterisch gehinderte Phenole organische Polymere von Zerstörung durch Licht und Wärme schützen.

Unbefriedigend ist bei den Verbindungen des Standes der Technik häufig die geringe Verträglichkeit mit Polyolefinen und anderen Kunststoffen, die Dauer der Schutzwirkung, die Eigenfarbe der Substanzen, die Neigung zur Flüchtigkeit und die thermische Zersetzung der Stabilisatoren beim Einarbeiten bei erhöhter Temperatur.

Der Erfindung lag die Aufgabe zugrunde, neue Stabilisatoren zur Verfügung zu stellen, welche die vorstehenden Nachteile nicht aufweisen.

Diese Aufgabe wird mit Hilfe der neuen Heterocyclen der Formel (I) gelöst. Dementsprechend betrifft die Erfindung Bicyclo-[3.3.1] nonanderivate der allgemeinen Formel (I) 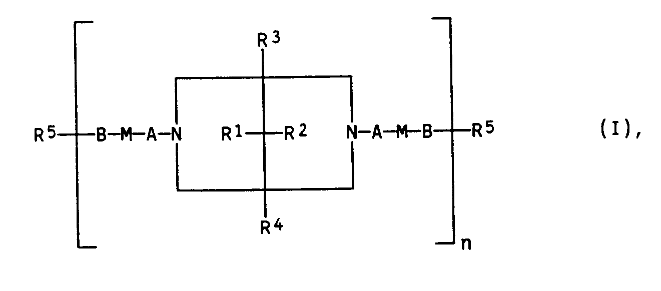
in der
- n: für die Zahl 1,
- R¹: für Wasserstoff,
- R²: für Wasserstoff oder Hydroxy oder der Rest 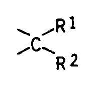 für 〉C=O ,
- R³ und R⁴: unabhängig voneinander für Phenyl, 1- oder 2-Naphthyl, wobei das Phenyl und Naphthyl durch 1, 2 oder 3 C₁- bis C₁₂-Alkyl, C₁- bis C₁₂-Alkoxy, Chlor, Brom, Fluor, Trifluormethyl oder Cyan substituiert sein können und wobei bei 2 und 3 Substituenten diese gleich oder verschieden sein können,
- A und B: unabhängig voneinander für eine chemische Bindung, C₁- bis C₂₂-Alkylen, Cycloalkylen 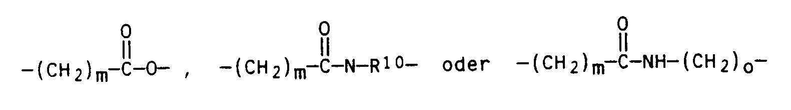 worin
- m: und o jeweils Zahlen von 1 bis 20 und
- R¹⁰: Wasserstoff, C₁-C₂₂-Alkyl, C₅-C₁₂-Cycloalkyl, C₇-C₁₈-Phenylalkyl, Phenyl oder C₂-C₂₂-Cyanalkyl bedeuten,
- M: für einen Rest der Formel 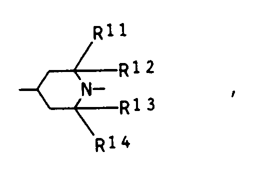 wobei M sowohl über das Stickstoffatom als auch über das Kohlenstoffatom an A gebunden sein kann, und
- R¹¹, R¹², R¹³ und R¹⁴: unabhängig voneinander C₁-C₄-Alkyl oder
- R¹¹ und R¹² und/oder R¹³ und R¹⁴: zusammen eine Tetra- oder Pentamethylengruppe bedeuten,
- R⁵: für Wasserstoff, Cyan, Hydroxy, 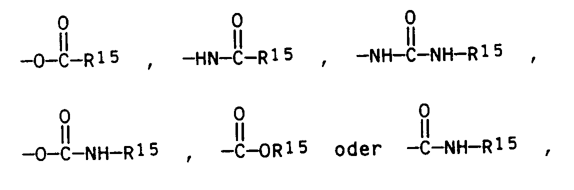 worin
- R¹⁵: Wasserstoff, C₁-C₂₂-Alkyl, C₅-C₁₂-Cycloalkyl, C₅-C₁₆-Phenylalkyl oder einen von Thiophen, Furan oder Pyridin abgeleiteten heterocyclischen Rest bedeutet oder -M-B-R⁵ für einen Rest der Formeln 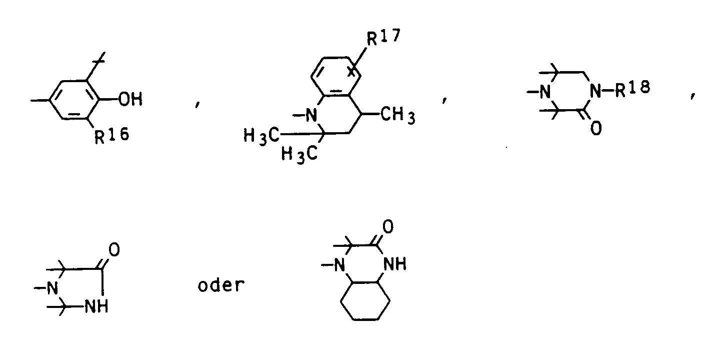 stehen, wobei in den Formeln
- R¹⁶: C₁-C₄-Alkyl,
- R¹⁷: Wasserstoff, C₁-C₄-Alkyl oder C₁-C₄-Alkoxy und
- R¹⁸: Wasserstoff oder C₁-C₁₂-Alkyl darstellen.

Die erfindungsgemäßen Verbindungen besitzen gegenüber Kunststoffen außerordentlich gute stabilisierende Eigenschaften. Sie haben keine Eigenfarbe, sind gut verträglich mit organischen Polymeren, haben einen niedrigen Dampfdruck und sind stabil gegen thermische Zersetzung.

Die erfindungsgemäßen Verbindungen eignen sich daher hervorragend zum Stabilisieren von Kunststoffen und Lacken gegen den Abbau durch Licht und Wärme. Sie sind auch wirksam als Metalldesaktivatoren.

R¹¹, R¹², R¹³ und R¹⁴ sind vorzugsweise Methyl.

Die Alkylreste R¹⁰, R¹⁵, R¹⁶, R¹⁷ und R¹⁸ können linear oder unverzweigt sein. Für diese Alkylreste sind im einzelnen z.B. zu nennen: Methyl, Ethyl, Propyl, iso-Propyl, Butyl, iso-Butyl, Pentyl, Hexyl, i-Octyl, Octyl, Nonyl, Decyl, Lauryl, Stearyl und Palmityl.

Cycloalkylreste für R¹⁰ und R¹⁵ sind beispielsweise Cyclopentyl, Cyclohexyl, Cycloheptyl, Cyclooctyl und Cyclododecyl.

R² steht vorzugsweise für Hydroxy.

Weiterhin sind Verbindungen der allgemeinen Formel (I) bevorzugt, bei denen der Rest 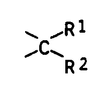
für eine Carbonylgruppe steht.

Für R¹⁵ sind C₁- bis C₆-Alkyl bevorzugt, z.B. Ethyl, Propyl, iso-Propyl, Butyl, iso-Butyl, Pentyl und n- und i-Hexyl. R¹⁸ steht vorzugsweise für Wasserstoff oder Methyl und insbesondere für tertiär-Butyl.

Phenylalkylreste für R¹⁰ und R¹⁵ sind beispielsweise Benzyl, Methylbenzyl, 2-Phenylethyl, 2- oder 3-Phenylpropyl und 2-, 3- oder 4-Phenylbutyl.

Als Alkylengruppen kommen für A und B C₁- bis C₂₂-Alkylen, vorzugsweise C₁- bis C₆-Alkylen in Betracht, wobei der Alkylenrest linear oder verzweigt sein kann.

A und B können außerdem für Reste der Formeln -(CH₂)ₘ-CO-N(R¹⁰)-(CH₂)ₘ-CO-O- und -(CH₂)ₘ-CO-NH-(CH₂)ₒ- stehen, wobei in den Formeln m = 1 bis 20, vorzugsweise 1 bis 3, o = 1 bis 20, vorzugsweise 1 bis 6, insbesondere 1 bis 3 und R¹⁰ Wasserstoff, Methyl oder Ethyl bedeuten.

Besonders bevorzugt sind für A und B -CH₂- und -CH₂-CH₂- und ganz besonders die direkte Bindung.

Besonders bevorzugt sind solche Verbindungen der Formel I, in der die Gruppierung A-M-B-R⁵ für 2,2,6,6-Tetramethylpiperidin-4-yl oder 1,2,2,6,6-Pentamethylpiperidin-4-yl steht.

Als Reste R³ und R⁴ kommen Phenyl, 1- und 2-Naphthyl in Betracht, wobei der Phenyl- und Naphthylrest 1, 2 oder 3 Substituenten tragen können. Als solche sind zu nennen: C₁- bis C₁₂-Alkyl, C₁- bis C₁₂-Alkoxy, Chlor, Brom, Fluor, Trifluormethyl und Cyan, wobei bei 2 oder 3 Substituenten diese gleich oder verschieden sein können.

Bevorzugt ist für R³ und R⁴ Phenyl, das gegebenenfalls 1- oder 2-fach substituiert ist. Als Reste sind für R³ und R⁴ z.B. im einzelnen zu nennen: Phenyl, 2-, 3- und 4-Tolyl, 3- und 4-Methoxyphenyl, 3- und 4-Ethoxyphenyl, 3- und 4-Chlorphenyl, 3- und 4-Bromphenyl, 3- und 4-Fluorphenyl, 3,4-Dimethylphenyl, 3- und 4-Trifluormethylphenyl, 4-tert.-Butylphenyl, 4-n-Butoxyphenyl, 4-Hexoxyphenyl, 4-Dodecyloxyphenyl und 3,4-Dimethoxyphenyl. Insbesondere ist unsubstituiertes Phenyl bevorzugt.

Von ganz besonderer Bedeutung ist das Bicyclo-[3.3.1]nonanderivat der Formel 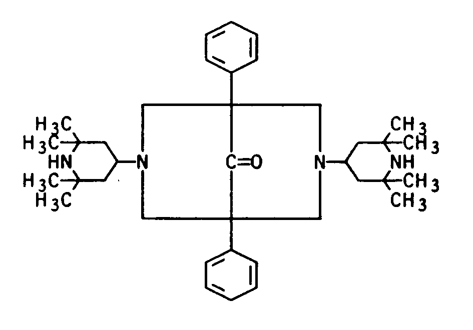
Verbindungen der allgemeinen Formel (I), in denen 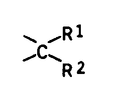
eine Carbonylgruppe ist, lassen sich nach an sich bekannten Verfahren durch Reaktion von Verbindungen der allgemeinen Formel (II) mit mindestens 4 Equivalenten Formaldehyd oder einer formaldehydbildenden Verbindung und mindestens 2 Equivalenten eines primären Amins der allgemeinen Formeln (III) bis (IX) herstellen

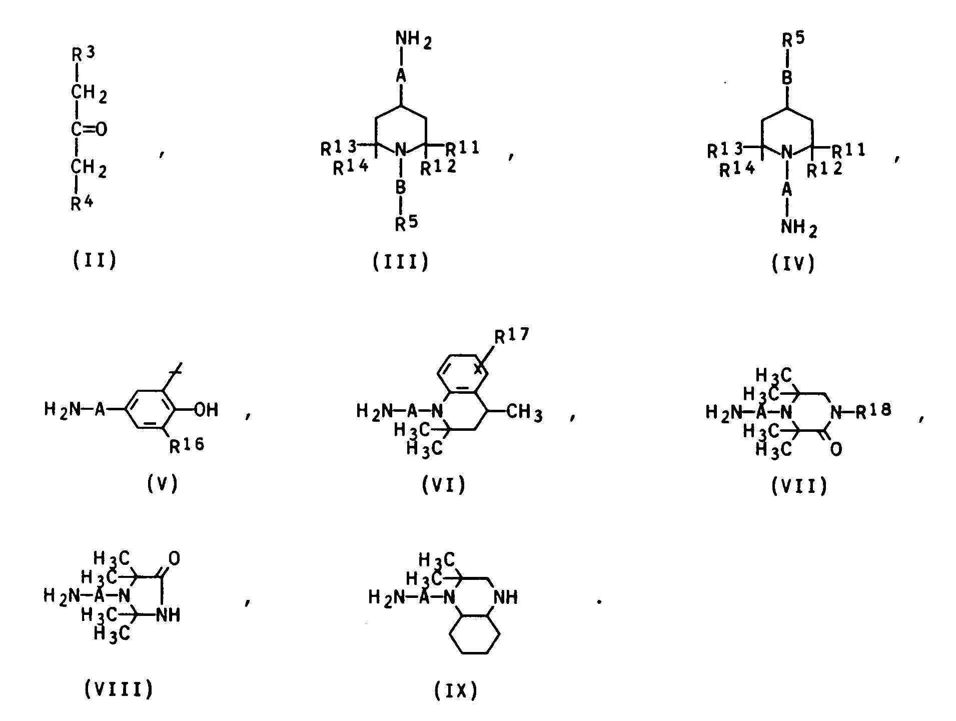

Die Herstellung ähnlicher Bicyclo-[3.3.1]-Derivate ist z.B. in J. Chem. Soc. 1951, 1706 beschrieben.

Vorzugsweise arbeitet man in einem Lösungsmittel. Bevorzugte Lösungsmittel sind Wasser und Alkohole, insbesondere Methanol und Ethanol.

In den Fällen, in denen 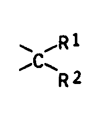
für eine Carbonylgruppe steht, kann diese durch Reduktionsmittel wie Wasserstoff oder Metallydride, z.B. Lithiumaluminiumhydrid und insbesondere Natriumborhydrid in die -CHOH-Gruppe überführt werden.

Die erfindungsgemäßen Verbindungen (I) können in Form der freien Basen, als Hydrate oder als Salze vorliegen. Geeignete Anionen stammen z.B. von anorganischen Säuren, Carbonsäuren und Sulfonsäuren. Von den Salzen sind die der Carbonsäuren und Sulfonsäuren bevorzugt.

Als anorganische Anionen kommen z.B. Chlorid, Bromid, Sulfat, Methosulfat, Tetrafluoroborat, Phosphat und Rhodanid in Betracht.

Carbonsäure-Anionen sind beispielsweise: Formiat, Acetat, Propionat, Hexanoat, Cyclohexanoat, Lactat, Stearat, Palmitat, Dodecylbenzoat, Benzoat, Acrylat, Methacrylat, Zitrat, Malonat und Succinat sowie Anionen von Polycarbonsäuren, wie Polyacrylsäure, Polymethacrylsäure und (Meth)Acrylsäure enthaltenden Copolymeren mit bis zu 3000 COOH-Gruppen.

Sulfonsäure-Anionen sind beispielsweise Benzolsulfonat, Tosylat und Methylsulfonat.

Den zu stabilisierenden Kunststoffen werden die Verbindungen (I) in einer Konzentration von 0,01 bis 5 Gew.% vorzugsweise von 0,02 bis 2 Gew.%, vor, während oder nach der Polymerbildung zugesetzt.

Zur Herstellung der Gemische aus den erfindungsgemäßen Verbindungen und den zu stabilisierenden Kunststoffen können alle bekannten Verfahren zum Einmischen von Stabilisierungsmitteln oder anderen Zusätzen in Polymere angewandt werden.

Neben den erfindungsgemäßen Verbindungen (I) können die stabilisierten Kunststoffe gegebenenfalls noch weitere Additive enthalten, beispielsweise Antioxidantien, zusätzliche Lichtstabilisierungsmittel, Metalldesaktivatoren, antistatische Mittel, flammhemmende Mittel, außerdem Pigmente und Füllstoffe.

Antioxidantien und Lichtstabilisatoren, die den Kunststoffen neben den erfindungsgemäßen Verbindungen (I) zugesetzt werden können, sind z.B. Verbindungen auf der Basis sterisch gehinderter Phenole, Schwefel und/oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxyethyl]-isocyanurat, 1,3,5-Tris-(2,6, di-methyl-3-hydroxy-4-tert.-butylbenzyl)-isocyanurat und Pentaerythrit-tetrakis-[β-(3,5-di-tert.-butyl-4-hydroxy-phenyl)-propionat] erwähnt.

Als phosphorhaltige Antioxidantien kommen z.B. Tris-(nonyl-phenyl)phosphit, Distearylpentaerythritdiphosphit, Tris-(2,4-di-tert.-butylphenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) erwähnt.

Weitere Antioxidantien und Lichtstabilisatoren, die zusammen mit den erfindungsgemäßen Verbindungen verwendet werden können, sind z.B. 2-(2'-Hydroxyphenyl)-benztriazole, 2-Hydroxybenzophenone, Phenylester von Hydroxybenzoesäuren, α-Cyanozimtsäurederivate, Nickelverbindungen und/oder Oxalsäuredianilide.

Als organische Polymere, die durch die erfindungsgemäßen Verbindungen stabilisiert werden können, kommen z.B. in Betracht:

Polymere von Mono- und Diolefinen, wie Polyethylen niedriger und hoher Dichte, lineares Polyethylen niedriger Dichte, Polypropylen, Polyisobutylen, Polybuten-1, Polyisopren, Polybutadien sowie Copolymerisate von Mono- oder Diolefinen und Mischungen der genannten Polymere;

Copolymerisate von Mono- oder Diolefinen mit weiteren Vinylmonomeren, z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol;
Copolymere aus Styrol oder α-Methylstyrol und Dienen oder Acrylderivaten, wie Styrol-Butadien, Styrol-Acrylnitril, Styrol-Ethylmethacrylat, Styrol-Butadien-Ethylacrylat, Styrol-Acrylnitril-Methacrylat.
ABS-, MBS- oder ähnliche Polymere;
Halogenhaltige Polymere, z.B. Polyvinylchlorid, Polyvinylfluorid, Polyvinylidenfluorid sowie deren Copolymere;
Polymere die sich von α,β-ungesättigten Säuren und deren Derivaten ableiten, wie Polyacrylate und Polymethacrylate, Polyacrylamide und Polyacrylnitrile;
Polymere, die sich von ungesättigten Alkoholen und Aminen oder von deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weitere organische Polymere, die mit den erfindungsgemäßen Verbindungen stabilisiert werden können, sind Industrielackierungen. Von diesen sind die Einbrennlackierungen, von denen wiederum die Fahrzeuglackierungen, vorzugsweise Zweischichtlackierungen, besonders hervorzuheben sind. Auch hier können die bereits genannten weiteren Antioxidantien und Lichtschutzmittel noch zugesetzt werden.

Die erfindungsgemäßen Verbindungen können dem Lack in fester oder gelöster Form zugegeben werden. Die gute Löslichkeit von (I) in den Lacksystemen ist dabei von besonderem Vorteil.

Die erfindungsgemäßen Verbindungen sind vor allem zur Stabilisierung von Polyolefinen, insbesondere von Ethylen- und Propylenpolymerisaten, von Polyurethanen und Lacken geeignet.

Die Erfindung soll durch die folgenden Beispiele weiter erläutert werden.

Die Ausbeuten sind in keinem Fall optimiert.

### Beispiel 1

315 g 1,3-Diphenylaceton, 468 g 2,2,6,6-Tetramethyl-4-amino-piperidin und 225 g Paraformaldehyd wurden in 1,2 l Ethanol 6 h gekocht. Nach dem Abkühlen wurde der ausgefallene Niederschlag abgesaugt, mit wenig Ethanol gewaschen und getrocknet. Man isolierte 563 g der Verbindung der Formel 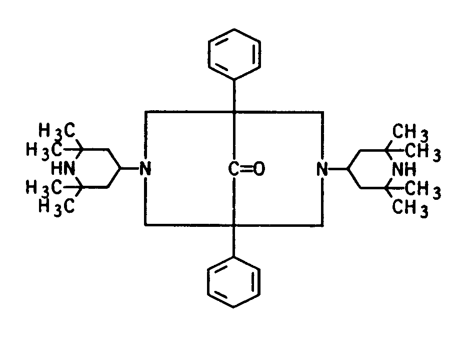
als farblosen Feststoff vom Schmp. 212 - 213 °C.

### Beispiel 2

14,3 g des Produkts aus Beispiel 1 wurden in wenig Methanol gelöst und mit 1 g Natriumborhydrid versetzt. Man erhitzte 2 h auf 50 °C, kühlte ab, saugte den ausgefallenen Niederschlag ab, wusch mit Wasser nach und trocknete. Man isolierte 9,7 g der Verbindung der Formel 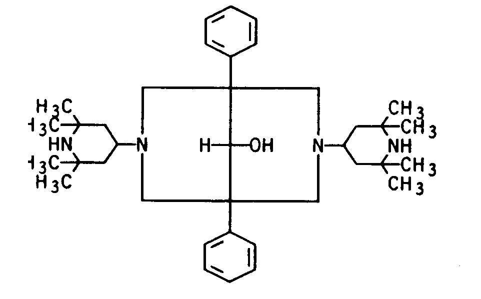
als farblosen Feststoff vom Schmp. 254 - 259 °C.

### Beispiel 3

Zu 28,6 g des Produkts aus Beispiel 1 gab man unter Eiskühlung eine Mischung aus 80 ml 98 %iger Ameisensäure und 15 ml Wasser und 6 g einer 30 %igen wäßrigen Formaldehydlösung. Man erhitzte 6 h auf 95 °C, gab nach dem Abkühlen 10 ml konzentrierte Salzsäure zu, engte ein und stellte mit 25 %iger wäßriger Natronlauge alkalisch. Das Wasser wurde von ausgefallenem Produkt abdekantiert. Dies wurde aus wenig Ethanol umkristallisiert. Man isolierte 10,3 g der Verbindung der Formel 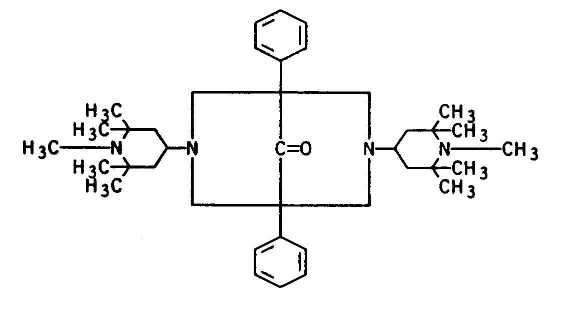
als farblosen Feststoff vom Schmp. 170 - 173 °C.

### Beispiel 4

Zu 9,4 g des Produkts aus Beispiel 3 in 250 ml Methanol gab man 1,75 g Natriumborhydrid und erhitzte 4,5 h auf 50 °C. Das Reaktionsgemisch wurde filtriert, in 500 ml Wasser eingetragen und der ausgefallene Niederschlag abgesaugt (6,0 g). Nach dem Umkristallisieren aus Acetonitril erhielt man 3,15 g der Verbindung der Formel 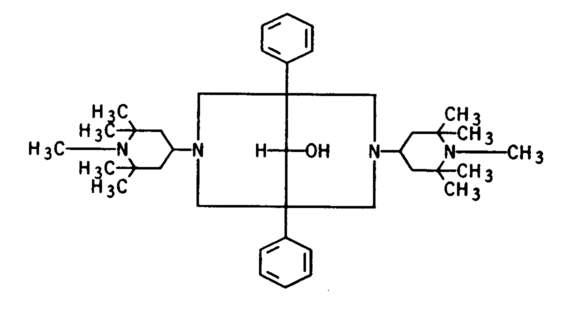
als farblosen Feststoff vom Schmp. 239 - 242 °C.

### Beispiel 5

12,5 ml mit Kaliumcarbonat gesättigtes Ethanol, 15 g Paraformaldehyd, 1,2 g Kaliumcarbonat und 42,5 g Acetoncyanhydrin wurden 2 Stunden bei 25 bis 30 °C gehalten und anschließend mit 85 %iger Phosphorsäure auf pH = 6 eingestellt. Dazu wurden 400 ml Ethanol und 71,5 g des Produkts aus Beispiel 1 gegeben und das Gemisch 9 h bei Siedetemperatur gehalten. Nach dem Abkühlen wurde der ausgefallene Niederschlag abgesaugt, mit 500 ml Wasser auf 80 °C erhitzt, heiß abgesaugt und getrocknet. Man erhielt 72 g der Verbindung der Formel 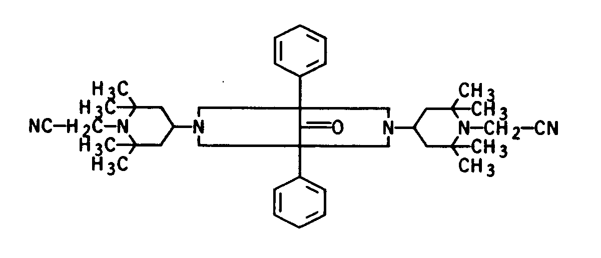
in Form des Halbhydrats vom Schmp. 230 - 233 °C.

### Beispiel 6

11,9 g 1,3-Di-(4'-methylphenyl)-aceton, 15,6 g 2,2,6,6-Tetramethyl-4-aminopiperidin und 7,5 g Paraformaldehyd wurden in 40 ml Ethanol 16 Stunden zum Rückfluß erhitzt. Die klare Lösung wurde am Rotationsverdampfer auf ca. 75 % das Volumen eingeengt. Das Konzentrat wurde im Eisbad gekühlt, der ausgefallene Niederschlag abgesaugt und mit wenig Methanol gewaschen. Man isolierte 14,4 g der Verbindung der Formel 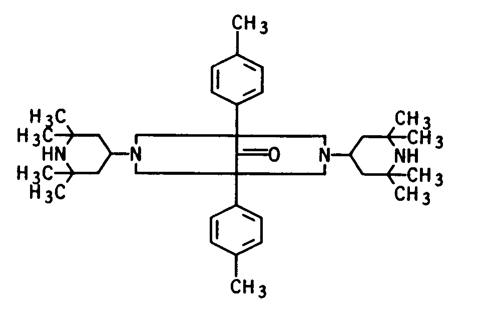
als farblosen Feststoff vom Schmp. 110 - 113 °C. Die Substanz enthält 0,25 Mol Wasser.

### Beispiel 7

38 g 1-β-Aminoethyl-2,2,6,6-tetramethyl-4-hydroxypiperidin (DE-A 32 08 570), 14,7 g 1,3-Diphenylaceton und 9 g Paraformaldehyd wurden in 150 ml Ethanol 18,5 Stunden gekocht. Nach dem Abkühlen wurde die Reaktionslösung bei Raumtemperatur langsam unter Rühren in 2 l Wasser getropft. Der ausgefallene Niederschlag wurde abgesaugt, mit Wasser gewaschen und an der Luft getrocknet. Nach dem Auskochen mit n-Heptan wurde der Rückstand aus Toluol unter Zusatz von Aktivkohle umkristallisiert. Man erhielt 22 g der Verbindung der Formel 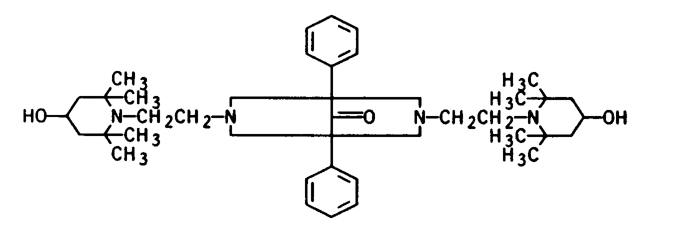
als farblosen Feststoff vom Schmp. 193 °C.

### Beispiel 8

a) 400 ml Ethanol, 678 g Cyanessigsäureethylester und 930 g 2,2,6,6-Tetramethyl-4-aminopiperidin wurden 7 Stunden gekocht und anschließend im Eisbad auf 10 °C abgekühlt. Der ausgefallene Niederschlag wurde abgesaugt und mit kaltem Essigsäureethylester gewaschen. Man erhielt 680 g der Verbindung der Formel 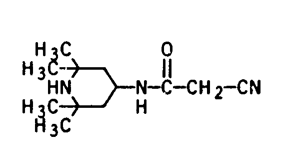 als farblosen Feststoff vom Schmp. 148 °C.
b) 112 g des Produkts aus Beispiel 8a) wurden unter Zusatz von 25 g Raney-Nickel und 100 g Ammoniak in 1000 ml Toluol bei 90 °C und einem Wasserstoffdruck von 200 bar bis zur Druckkonstanz hydriert. Nach dem Abfiltrieren wurde eingeengt und der Rückstand unter Zusatz eines hochsiedenden Propylenoxid-Ethylenoxid-Blockpolymeren mit einem mittleren Molekulargewicht von ca. 6000 (Pluriol® PE 6400 der Fa. BASF) destilliert. Man erhielt 80 g der Verbindung der Formel 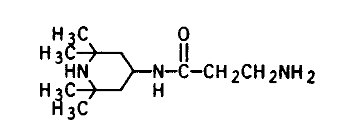 als farbloses Öl vom Siedepunkt 155 °C bei 0,4 Torr, das nach längerem Stehen erstarrt.
c) 33 g des Produkts aus Beispiel 8b), 13,6 g 1,3-Diphenylaceton und 7,8 g Paraformaldehyd wurden in 150 ml Ethanol 20 Stunden gekocht. Anschließend wurde das Lösungsmittel im Wasserstrahlvakuum abdestilliert, der klebrige Rückstand in einer Mischung aus 200 g Wasser und 10 g konzentrierter Schwefelsäure gelöst und mit 200 ml Essigsäureethylester ausgeschüttelt. Die Wasserphase wurde mit etwas Aktivkohle versetzt, filtriert und mit konzentrierter Natronlauge alkalisch gestellt. Der ausgefallene Niederschlag wurde abgesaugt, an der Luft getrocknet und aus einem Gemisch aus Methylcyclohexan und Toluol umkristallisiert. Man erhielt 13,9 g der Verbindung der Formel 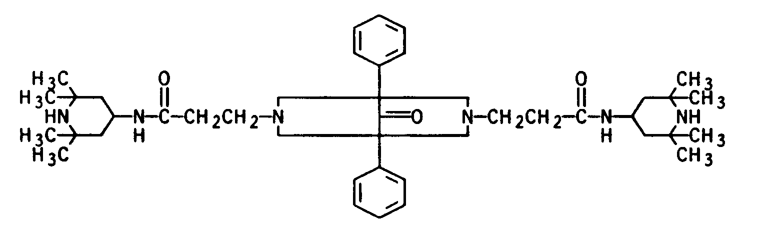 vom Schmp. 88 °C.

## Patentansprüche

1. Heterocyclische Bicyclo-[3.3.1]nonanderivate der allgemeinen Formel (I) 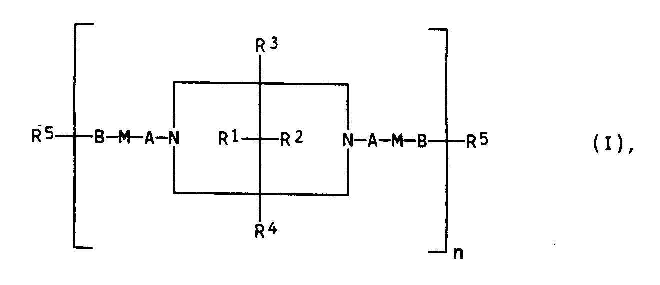 in der
n für die Zahl 1,
R¹ für Wasserstoff,
R² für Wasserstoff oder Hydroxy oder der Rest 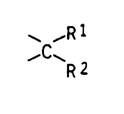 für 〉C=O ,
R³ und R⁴ unabhängig voneinander für Phenyl, 1- oder 2-Naphthyl, wobei das Phenyl und Naphthyl durch 1, 2 oder 3 C₁- bis C₁₂-Alkyl, C₁- bis C₁₂-Alkoxy, Chlor, Brom, Fluor, Trifluormethyl oder Cyan substituiert sein können und wobei bei 2 und 3 Substituenten diese gleich oder verschieden sein können,
A und B unabhängig voneinander für eine chemische Bindung, C₁- bis C₂₂-Alkylen, Cycloalkylen, 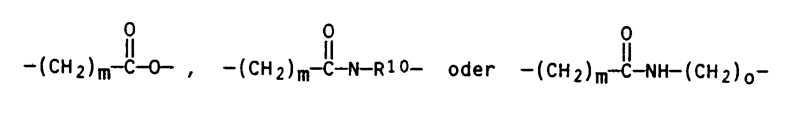 worin
m und o jeweils Zahlen von 1 bis 20 und
R¹⁰ Wasserstoff, C₁-C₂₂-Alkyl, C₅-C₁₂-Cycloalkyl, C₇-C₁₈-Phenylalkyl, Phenyl oder C₂-C₂₂-Cyanalkyl bedeuten,
M für einen Rest der Formel 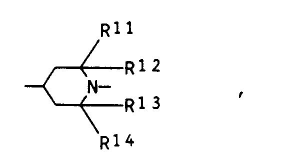 wobei M sowohl über das Stickstoffatom als auch über das Kohlenstoffatom an A gebunden sein kann, und
R¹¹, R¹², R¹³ und R¹⁴ unabhängig voneinander C₁-C₄-Alkyl oder
R¹¹ und R¹² und/oder R¹³ und R¹⁴ zusammen eine Tetra- oder Pentamethylengruppe bedeuten,
R⁵ für Wasserstoff, Cyan, Hydroxy, 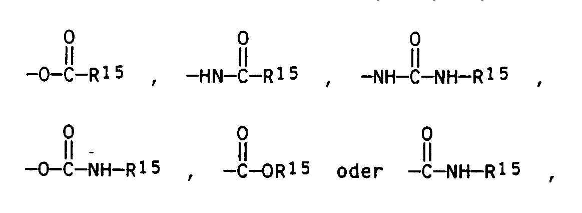 worin
R¹⁵ Wasserstoff, C₁-C₂₂-Alkyl, C₅-C₁₂-Cycloalkyl, C₅-C₁₆-Phenylalkyl oder einen von Thiophen, Furan oder Pyridin abgeleiteten heterocyclischen Rest bedeutet, oder -M-B-R⁵ für einen Rest der Formeln 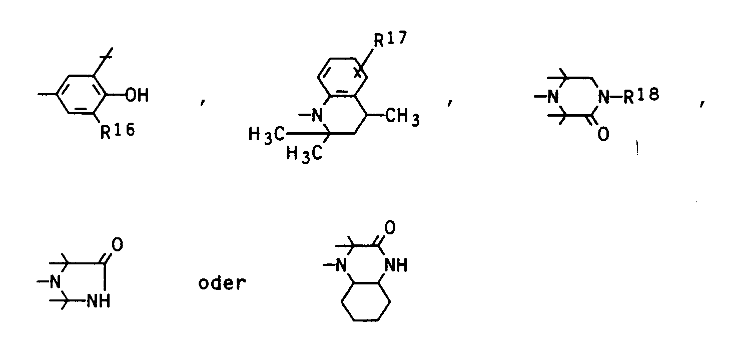 stehen, wobei in den Formeln
R¹⁶ C₁-C₄-Alkyl,
R¹⁷ Wasserstoff, C₁-C₄-Alkyl oder C₁-C₄-Alkoxy und
R¹⁸ Wasserstoff oder C₁-C₁₂-Alkyl darstellen.

2. Bicyclononanderivate gemäß Anspruch 1, dadurch gekennzeichnet, daß R¹¹, R¹², R¹³ und R¹⁴ Methyl sind.

3. Bicyclononanderivate gemäß Anspruch 1 oder 2, dadurch gekennzeichnet, daß R² für Hydroxy oder der Rest 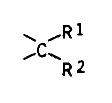 für eine Carbonylgruppe stehen.

4. Bicyclononanderivate gemäß Anspruch 1, 2 oder 3, dadurch gekennzeichnet, daß R³ und R⁴ für Phenyl stehen.

5. Bicyclononanderivate gemäß den Ansprüchen 1 bis 4, dadurch gekennzeichnet, daß A und B für eine direkte Bindung stehen.

6. Bicyclononanderivate gemäß den Ansprüchen 1 bis 4, dadurch gekennzeichnet, daß A für -CH₂-CH₂- steht.

7. Bicyclononanderivate gemäß den Ansprüchen 1 bis 5, dadurch gekennzeichnet, daß die Gruppierung A-M-B-R⁵ für 2,2,6,6-Tetramethylpiperidin-4-yl oder 1,2,2,6,6-Pentamethylpiperidin-4-yl steht.

8. Verwendung der Bicyclononanderivate gemäß den Ansprüchen 1 bis 7 zum Stabilisieren von Kunststoffen und Lacken.

9. Verwendung der Bicyclononanderivate gemäß den Ansprüchen 1 bis 7 zum Stabilisieren von Polyurethanen.

10. Verwendung der Bicyclononanderivate gemäß den Ansprüchen 1 bis 7 zum Stabilisieren von Polyolefinen.

11. Verwendung der Bicyclononanderivate gemäß den Ansprüchen 1 bis 7 zum Stabilisieren von Polyamiden.

12. Verwendung der Bicyclononanderivate gemäß den Ansprüchen 1 bis 7 als Licht- und Wärmeschutz oder als Metallionendesaktivator.

13. Verwendung der Bicyclononanderivate gemäß den Ansprüchen 1 bis 7 zum Stabilisieren von ABS.

14. Stabilisierte Kunststoffe und Lacke, enthaltend Bicyclononanderivate gemäß den Ansprüchen 1 bis 7.

## Claims

1. A heterocyclic bicyclo[3.3.1]nonane derivative of the general formula (I) 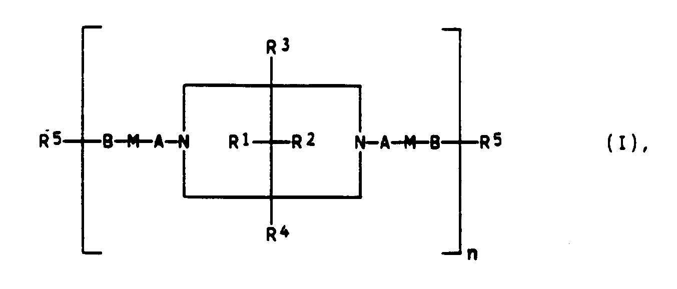 where
n is 1,
R¹ is hydrogen,
R² is hydrogen or hydroxyl, or the radical 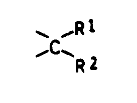 is 〉C=O ,
R³ and R⁴ are each independently of the other phenyl or 1- or 2-naphthyl, which phenyl or naphthyl may each be substituted by 1, 2 or 3 C₁-C₁₂-alkyls, C₁-C₁₂-alkoxys, chlorines, bromines, fluorines, trifluoromethyls or cyanos, in the case of 2 or 3 substituents these substituents being identical or different,
A and B are each independently of the other a chemical bond, C₁-C₂₂-alkylene, cycloalkylene 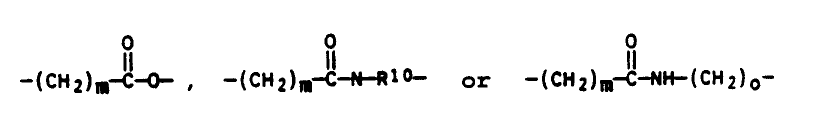 where
m and o are each from 1 to 20 and
R¹⁰ is hydrogen, C₁-C₂₂-alkyl, C₅-C₁₂-cycloalkyl, C₇-C₁₈-phenylalkyl, phenyl or C₂-C₂₂-cyanoalkyl,
M is a radical of the formula 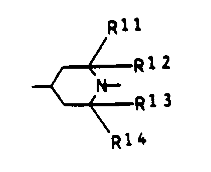 which may be bonded to A not only via the nitrogen atom but also via the carbon atom, and
R¹¹, R¹², R¹³ and R¹⁴ are each independently of the others C₁-C₄-alkyl, or
R¹¹ and R¹² and/or R¹³ and R¹⁴ are each together tetramethylene or pentamethylene,
R⁵ is hydrogen, cyano, hydroxyl, 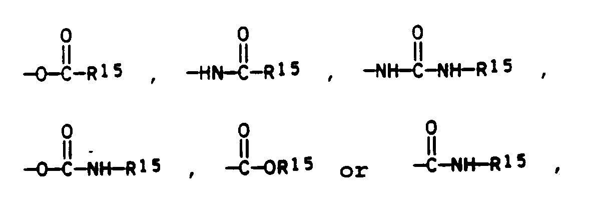 where
R¹⁵ is hydrogen, C₁-C₂₂-alkyl, C₅-C₁₂-cycloalkyl, C₅-C₁₆-phenylalkyl or a heterocyclic radical derived from thiophene, furan or pyridine, or -M-B-R⁵ is a radical of the formula 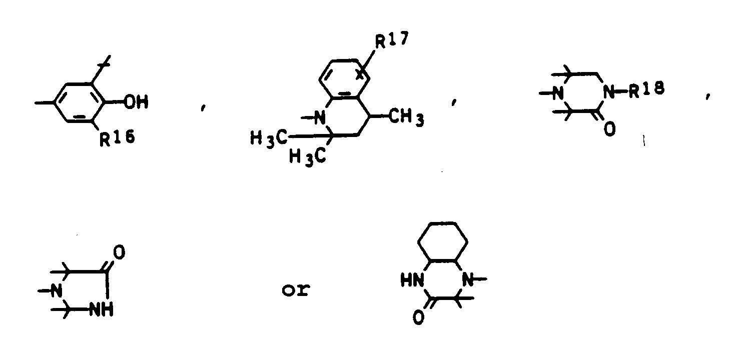 where
R¹⁶ is C₁-C₄-alkyl,
R¹⁷ is hydrogen, C₁-C₄-alkyl or C₁-C₄-alkoxy and
R¹⁸ is hydrogen or C₁-C₁₂-alkyl.

2. A bicyclononane derivative as claimed in claim 1, wherein R¹¹, R¹², R¹³ and R¹⁴ are each methyl.

3. A bicyclononane derivative as claimed in claim 1 or 2, wherein R² is hydroxyl or the radical 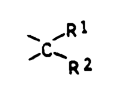 is carbonyl.

4. A bicyclononane derivative as claimed in claim 1, 2 or 3, wherein R³ and R⁴ are each phenyl.

5. A bicyclononane derivative as claimed in any of claims 1 to 4, wherein A and B are each a direct bond.

6. A bicyclononane derivative as claimed in any of claims 1 to 4, wherein A is -CH₂-CH₂-.

7. A bicyclononane derivative as claimed in any of claims 1 to 5, wherein A-M-B-R⁵ is 2,2,6,6-tetramethylpiperidin-4-yl or 1,2,2,6,6-pentamethylpiperidin-4-yl.

8. The use of a bicyclononane derivative as claimed in any of claims 1 to 7 for stabilizing plastics and coatings.

9. The use of a bicyclononane derivative as claimed in any of claims 1 to 7 for stabilizing polyurethanes.

10. The use of a bicyclononane derivative as claimed in any of claims 1 to 7 for stabilizing polyolefins.

11. The use of a bicyclononane derivative as claimed in any of claims 1 to 7 for stabilizing polyamides.

12. The use of a bicyclononane derivative as claimed in any of claims 1 to 7 as a light or thermal stabilizer or as a metal ion deactivator.

13. The use of a bicyclononane derivative as claimed in any of claims 1 to 7 for stabilizing ABS.

14. A stabilized plastic or coating comprising a bicyclononane derivative as claimed in any of claims 1 to 7.

## Revendications

1. Dérivés hétérocycliques du bicyclo-[3.3.1]nonane de la formule générale (I) 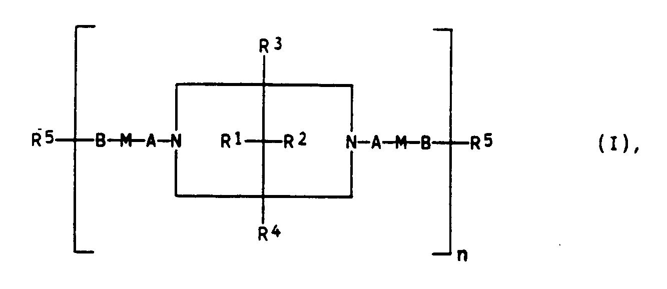 dans laquelle représentent
n le nombre 1,
R¹ l'hydrogène,
R² l'hydrogène ou le radical hydroxyle ou le radical 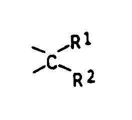 le radical 〉C=O ,
R³ et R⁴ , indépendamment l'un de l'autre, un radical phényle, 1- ou 2-naphtyle, ou les groupes phényle et naphtyle peuvent être substitues par 1, 2 ou 3 radicaux alkyle en C₁-C₁₂, alcoxy en C₁-C₁₂, atomes de chlore, de brome, de fluor, radicaux trifluorométhyle ou cyano et où dans le cas de la présence de 2 et de 3 substituants, ceux-ci peuvent être identiques ou différents,
A et B , indépendamment l'un de l'autre, une liaison chimique, un radical cycloalkylène, alkylène en C₁ à C₂₂, 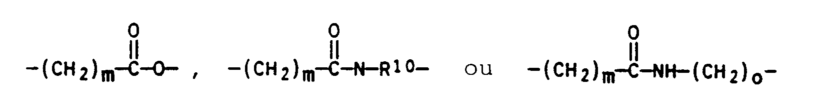 où
m et o sont chacun des nombres de 1 à 20 et
R¹⁰ l'hydrogène, un radical alkyle en C₁ à C₂₂, cycloalkyle en C₅ à C₁₂, phénylalkyle en C₇ à C₁₈, phényle ou cyanalkyle en C₂ à C₂₂,
M un reste de la formule 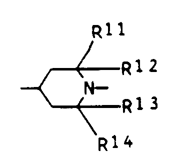 où M peut être lié à A aussi bien par l'intermédiaire d'un atome d'azote qu'également par l'intermédiaire d'un atome de carbone et
R¹¹, R¹², R¹³ et R¹⁴ , indépendamment l'un de l'autre, des radicaux alkyle en C₁ à C₄ ou bien
R¹¹ et R¹² et/ou R¹³ et R¹⁴ forment ensemble un groupe tétra- ou pentaméthylène,
R⁵ l'hydrogène, un radical cyano, hydroxyle, 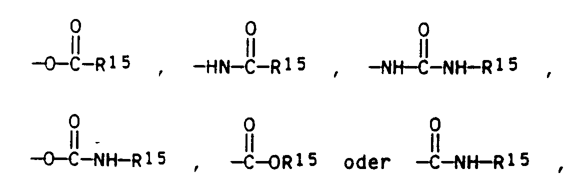 où
R¹⁵ désigne l'hydrogène, un radical alkyle en C₁ à C₂₂, cycloalkyle en C₅ à C₁₂, phénylalkyle en C₅ à C₁₆, ou un radical hétérocyclique qui dérive du thiophène, du furanne ou de la pyridine, ou bien -M-B-R⁵ représente un reste des formules 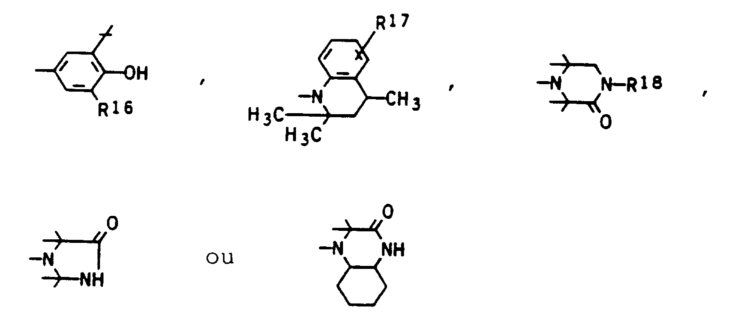 où dans les formules représentent
R¹⁶ un radical alkyle en C₁ à C₄,
R¹⁷ l'hydrogène, un radical alkyle en C₁ à C₄ ou alcoxy en C₁ à C₄ et
R¹⁸ l'hydrogène ou un radical alkyle en C₁ à C₁₂.

2. Dérivés du bicyclononane suivant la revendication 1, caractérisés en ce que R¹¹, R¹², R¹³ et R¹⁴ représentent des radicaux méthyle.

3. Dérivés du bicyclononane suivant la revendication 1 ou 2, caractérisés en ce que R² représente le radical hydroxyle ou le reste 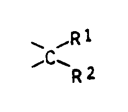 représente un radical carbonyle.

4. Dérivés du bicyclononane suivant la revendication 1, 2 ou 3, caractérisés en ce que R³ et R⁴ représentent des radicaux phényle.

5. Dérivés du bicyclononane suivant les revendications 1 à 4, caractérisés en ce que A et B représentent une liaison directe.

6. Dérivés du bicyclononane suivant les revendications 1 à 4, caractérisés en ce que A représente le reste -CH₂-CH₂-.

7. Dérivés du bicyclononane suivant les revendications 1 à 5, caractérisés en ce que le groupement A-M-B-R⁵ représente le radical 2,2,6,6-tétraméthylpipéridine-4-yle ou le radical 1,2,2,6,6-pentaméthylpipéridine-4-yle.

8. Utilisation des dérivés du bicyclononane suivant les revendications 1 à 7 pour la stabilisation de matières plastiques et de peintures ou laques.

9. Utilisation de dérivés du bicyclononane suivant les revendications 1 à 7 pour la stabilisation de polyuréthannes.

10. Utilisation de dérivés du bicyclononane suivant les revendications 1 à 7 pour la stabilisation de polyoléfines.

11. Utilisation de dérivés du bicyclononane suivant les revendications 1 à 7 pour la stabilisation de polyamides.

12. Utilisation des dérivés du bicyclononane suivant les revendications 1 à 7, à titre d'agents de protection contre la lumière et la chaleur, ou à titre de désactivateurs d'ions de métaux.

13. Utilisation des dérivés du bicyclononane suivant les revendications 1 à 7 pour la stabilisation de l'ABS.

14. Peintures ou laques et matières plastiques, stabilisées, contenant des dérivés du bicyclononane suivant les revendications 1 à 7.
